(19) Europäisches Patentamt
European Patent Office
Office européen des brevets

(11) Veröffentlichungsnummer : **0 037 003**
**B1**

(12) # EUROPÄISCHE PATENTSCHRIFT

(45) Veröffentlichungstag der Patentschrift : **24.08.83**

(51) Int. Cl.³ : **C 07 C 19/08**, C 07 C 17/04

(21) Anmeldenummer : **81101974.4**

(22) Anmeldetag : **17.03.81**

(54) **2,3-Dichlor-2-trifluormethyl-1,1,1,3,4,4,5,5,5-nonafluorpentan und Verfahren zu seiner Herstellung.**

(30) Priorität : **28.03.80 DE 3012005**

(43) Veröffentlichungstag der Anmeldung : **07.10.81 Patentblatt 81/40**

(45) Bekanntmachung des Hinweises auf die Patenterteilung : **24.08.83 Patentblatt 83/34**

(84) Benannte Vertragsstaaten : **BE DE FR GB IT NL**

(56) Entgegenhaltungen :

**HOUBEN-WEYL : « Methoden der Organischen Chemie », 4. Auflage, Band V/3. Halogenverbindungen, 1962. Georg Thieme Verlag, Seiten 532-542 Stuttgart, DE.**

**JOURNAL OF THE AMERICAN CHEMICAL SOCIETY, Band 84, 20. August 1962, Seiten 3148-3153 Easton, U.S.A. J.F. HARRIS JR. : « The free-radical addition of trifluoromethanesulfenyl chloride to haloölefins »**

(73) Patentinhaber : **HOECHST AKTIENGESELLSCHAFT**
***Postfach 80 03 20***
**D-6230 Frankfurt am Main 80 (DE)**

(72) Erfinder : **von Halasz, Sigmar-Peter, Dr.**
***Feldbergstrasse 50***
**D-6233 Kelkheim (Taunus) (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 037 003 B1**

Jouve, 18, rue St-Denis, 75001 Paris, France

# 2,3-Dichlor-2-trifluormethyl-1,1,1,3,4,4,5,5,5-nonafluorpentan und Verfahren zu seiner Herstellung

Die vorliegende Erfindung betrifft die neue Verbindung 2,3-Dichlor-2-trifluormethyl-1,1,1,3,4,4,5,5,5-nonafluorpentan (I) und ein Verfahren zu seiner Herstellung durch Photochlorierung von Perfluor-2-methyl-2-penten (II).

Durch Dimerisierung von Hexafluorpropen ist Perfluor-2-methyl-2-penten leicht zugänglich. Seine Umsetzung mit elementarem Fluor ist aus der DE-OS 23 32 088 bekannt und führt zum Perfluor-2-methylpentan, einer hochstabilen perhalogenierten Verbindung, die für viele Zwecke eingesetzt werden kann.

Nachteilig an diesem bekannten Verfahren ist der Einsatz von nicht leicht handhabbarem, kostspieligem elementarem Fluor.

Es bestand daher die Aufgabe, ausgehend von Perfluor-2-methyl-penten (II) ohne Verwendung von elementarem Fluor eine hochstabile perhalogenierte Verbindung herzustellen.

Es wurde nun gefunden, daß auch das bei der Umsetzung von II mit Chlor anfallende Produkt (I) sich durch hohe thermische und chemische Stabilität auszeichnet.

Es ist bekannt, daß elementares Chlor unter der Einwirkung von energiereichem Licht an fluorierte Olefine angelagert werden kann (Houben-Weyl, 4. Aufl. Band V/3, 540-542).

Mit höherem Fluorgehalt und zunehmendem Raumbedarf der vorhandenen Substituenten wird jedoch die Chloranlagerung immer schwieriger. So ist nach Untersuchungen von R. N. Haszeldine et al. (Soc. 1956, 69) für die Addition von Chlor an Perfluorbuten-(2) selbst bei Verwendung eines 20 %igen Chlorüberschusses eine Reaktionsdauer von 3 Tagen erforderlich, um eine Ausbeute von 88 % zu erzielen, obwohl in diesem Fall jedes Kohlenstoffatom an der Doppelbindung nur einen einzigen Perfluormethyl-Rest trägt. Es war daher zu erwarten, daß die Doppelbindung des Perfluor-(2-methyl-2-pentens) mit drei Perfluoralkyl-Substituenten noch träger mit Chlor reagiert.

Die Verbindung I stellt eine farblose Flüssigkeit vom Siedepunkt 112,5 °C dar. Sie besitzt folgende Konstitutionsformel

$$(CF_3)_2CCl—CClF—C_2F_5$$

Das erfindungsgemäße Verfahren zur Herstellung von 2,3-Dichlor-2-trifluormethyl-1,1,1,3,4,4,5,5,5-nonafluorpentan (I) ist dadurch gekennzeichnet, daß man Perfluor-2-methyl-2-penten (II) mit elementarem Chlor in Gegenwart von energiereichem Licht und in flüssigem 2,3-Dichlor-2-trifluormethyl-1,1,1,3,4,4,5,5,5-nonafluorpentan als Lösungsmittel bei Temperaturen von 20-300 °C umsetzt.

Das erfindungsgemäße Verfahren kann kontinuierlich nach Art bekannter Reaktionen ausgestaltet werden. Ein Feststoffkatalysator ist dabei nicht erforderlich. Bevorzugt ist bei diesem Verfahren ein Temperaturbereich von 50 bis 200 °C.

Man arbeitet in flüssiger Phase, chargenweise oder kontinuierlich. Bei Chargenbetrieb wird die Verbindung II, durch ein Lösungsmittel verdünnt, vorgelegt und anschließend elementares Chlor eingeleitet. Als Lösungsmittel lassen sich Halogenkohlenwasserstoffe mit hohem Siedepunkt, insbesondere das Endprodukt des Verfahrens (I), einsetzen. Die Notwendigkeit, eine flüssige Phase aufrecht zu erhalten, begrenzt in diesem Fall die Reaktionstemperatur bei Atmosphärendruck. Jedoch kann auch unter erhöhtem Druck gearbeitet werden, wobei die Raum/Zeit-Ausbeute zunimmt.

Bevorzugt ist es jedoch, wenn man kontinuierlich arbeitet und in vorgelegtes (I) gleichzeitig Chlor und die Verbindung (II) einleitet. Dabei soll (II) sieden oder bei einer Temperatur nahe dem Siedepunkt gehalten werden. Bevorzugt sind Temperaturen von 50 bis 200 °C.

Unter energiereichem Licht wird hier Strahlung verstanden, die in der Lage ist, Chlormoleküle in Chloratome zu zerlegen, insbesondere sichtbares Licht und ultraviolettes Licht im Bereich von 450 bis 260 nm.

Das erfindungsgemäße Verfahren verläuft nach folgender Gleichung

$$(CF_3)_2C = CFCF_2CF_3 + Cl_2 \longrightarrow (CF_3)_2CClCClFCF_2CF_3$$

Das eingesetzte Perfluor-2-methyl-2-penten (II) wird in technischer Reinheit, zweckmäßigerweise in wasserfreier Form verwendet; es läßt sich in leichter Weise durch gezielte Oligomerisierung von technisch verfügbarem Hexafluorpropen herstellen (z. B. gemäß Th. Martini und S. P. v. Halasz, Tetrahedron Letters *24*, 2129-32 (1974)). Elementares Chlor wird einer handelsüblichen Sthalflasche entnommen und zweckmäßigerweise in wasserfreier, Form eingesetzt, d. h. beispielsweise mit konzentrierter Schwefelsäure getrocknet.

Der Umsatz an Perfluor-2-methyl-2-penten beträgt bei Siedetemperatur und Atmosphärendruck je nach der Strahlungsintensität etwa 0,5 bis 4,0 Mol/l Flüssigphase und Stunde.

Chlor wird im allgemeinen ohne Verdünnung zugegeben; die Menge an Chlor liegt dabei im allgemeinen zwischen 0,5 und 4,5 Mol/l Flüssigphase und Stunde. Die Chlormenge soll der Menge des gleichzeitig und kontinuierlich zugegebenen Perfluor-2-methyl-2-pentens mindestens äquivalent sein. Bevorzugt ist ein geringer Chlorüberschuß. Das Molverhältnis (II) : Chlor liegt vorzugsweise zwischen 1 : 1 und 1 : 1,2, insbesondere zwischen 1 : 1 und 1 : 1,05.

Durch den Überschuß an Chlor läßt sich ein quantitativer Umsatz an Perfluor-2-methyl-2-penten erreichen. Größere Überschüsse an Chlor sind zwar möglich, jedoch nicht zweckmäßig, da bei der Benutzung eines wirksamen Kondensators die Gefahr besteht, daß unumgesetztes, am Kondensator verflüssigtes, in den Bestrahlungsreaktor zurückfließendes Chlor die Temperatur im Reaktor senkt und somit zu einer Verringerung der Reaktionsgeschwindigkeit führt. Ein Unterschuß an Chlor ist zwar möglich, führt aber zu einer Verringerung des Umsatzes und einer Erhöhung des Aufwandes bei der Aufarbeitung. Aus dem Reaktionsprodukt wird überschüssiges Chlor entweder herausgewaschen (mit wäßrigem Alkalihydroxid oder -thiosulfat) oder zweckmäßigerweise durch destillative Aufarbeitung abgetrennt.

Im allgemeinen wird das erfindungsgemäße Verfahren ohne den Zusatz eines Inertgases durchgeführt. Eine Verdünnung der gasförmigen Einsatzprodukte mit Stickstoff oder einem anderen Inertgas ist zwar möglich, bringt aber keine Vorteile.

Besonders bevorzugt für das erfindungsgemäße Verfahren sind Reaktionstemperaturen in Bereich von 60 bis 120°, insbesondere im Bereich von 80 bis 115 °C. Diese Temperaturen lassen sich bei chargenweisem Arbeiten durch Druckerhöhung, bei kontinuierlicher Zugabe durch Aufheizen des Reaktionsmediums leicht einhalten. Als Reaktormaterial wird vorzugsweise lichtdurchlässiges Glasmaterial, z. B. Borsilikat-Glas, verwendet.

Die Verweilzeit der niedrig siedenden Ausgangsprodukte und des höhersiedenden Endprodukts im Reaktor ist nicht kritisch ; sie kann beispielsweise für Produkt I mehrere Stunden oder Tage betragen. In keinem Fall wird dabei eine Beeinträchtigung der Rohprodukt-Zusammensetzung beobachtet. Andererseits begrenzen wirtschaftliche Erwägungen die Verweilzeit nach oben, da ja eine möglichst günstige Raum-Zeit-Ausbeute über eine lange Versuchsdauer angestrebt wird. Bei kontinuierlicher Arbeitsweise wird man die Zugabe der eindosierten Ausgangsprodukte und die Abnahme des Endproduktes so steuern, daß der Spiegel der flüssigen Phase im Bestrahlungsgefäß etwa konstant bleibt. Bei der kontinuierlichen Fahrweise kann überschüssig eingesetztes Chlor durch eine destillative Aufarbeitung zurückgewonnen und unmittelbar in den Reaktor zurückgeführt werden. Neben der hohen Ausnutzung der eingesetzten Stoffe ist dabei auch die äußerst geringe Menge von Abwasser und Abgas vorteilhaft.

Die erfindungsgemäße Chlorierung findet im allgemeinen bei Normaldruck statt, erlaubt jedoch auch die Anwendung von Unterdruck oder Überdruck. So kann man beispielsweise bei Drucken von 1 bis 50 bar, vorzugsweise bei 1 bis 10 bar, insbesondere bei 1 bis 3 bar arbeiten. Insbesondere zur Erzielung höherer Raum-Zeit-Ausbeuten ist die Anwendung von Überdruck vorzuziehen.

Beim erfindungsgemäßen Verfahren läßt sich leicht ein Umsatz an Perfluor-2-methyl-2-penten von über 99 % erreichen. Die Ausbeuten an 2,3-Dichlor-2-trifluormethyl-nonafluorpentan (I) liegen aufgrund der hohen Selektivität des erfindungsgemäßen Verfahrens bei ca. 98 % der Theorie. In Mengen von 0,1 bis 2 % können durch radikalische Dimerisierung Produkte mit der Summenformel $C_{12}Cl_2F_{24}$ entstehen. Diese Produkte besitzen unterschiedliche Konstitution und sieden über 200 °C. Daher gestaltet sich die destillative Aufarbeitung von (I) einfach. Es reicht z. B aus, das Rohprodukt destillativ aufzuarbeiten. Dabei lassen sich die hochsiedenden Nebenprodukte, Chlor und die niedrigsiedende Ausgangskomponente (II) entfernen.

Die nach dem erfindungsgemäßen Verfahren leicht zugängliche Verbindung I stellt aufgrund ihrer thermischen und chemischen Stabilität, ihrer Unbrennbarkeit sowie ihrer besonderen physikalischen, beispielsweise ihrer dielektrischen, Eigenschaften ein wertwolles Produkt für die Anwendung als Hydraulikflüssigkeit, Dielektrikum und Sicherheitskältemittel, insbesondere für Turbinenkompressoren, dar. Ebenso eignet sie sich als Wärmeüberträger in Wärmepumpen, als Arbeitsmedium in Expansionsmaschinen oder als Zusatzkomponente in Feuerlöschmitteln. Weiterhin kann 2,3-Dichlor-2-trifluormethyl-nonafluorpentan (I) als inertes Lösemittel oder Reaktionsmedium, beispielsweise bei Halogenierungen, verwendet werden. Ferner eignet sich I als schonendes Löse- und Entfettungsmittel in der technischen Reinigung, z. B. von Werkstoffen und elektrischen Bauteilen, oder auch in der chemischen Reinigung von Textilien oder als Zusatzkomponente für bereits verwendete Reinigungsmittel.

Dabei ist das hohe Molekulargewicht von 371, das einen hohen Siedepunkt und einen relativ niedrigen Dampfdruck bedingt, sowie der niedrige Erstarrungspunkt von – 134 °C von Vorteil. Die Dichte beträgt bei 20 °C 1,830 g/cm$^3$. Hervorzuheben ist der unerwartet weite Temperaturbereich der flüssigen Phase von I, der sich bei Normaldruck von + 112,5 bis – 134 °C erstreckt und der damit teilweise beträchtlich größer ist als der Flüssigphasen-Bereich anderer technisch verwendeter halogenierter Kohlenwasserstoffe. Aufgrund dieser besonderen physikalischen Eigenschaften, aber auch im Hinblick auf das Viskositätsverhalten und die gute Mischbarkeit mit anderen Chlorfluorkohlenwasserstoffen, eignet sich I auch als Schmiermittel, insbesondere auf dem Sektor der Tieftemperatur-Schmierung in Kälte- und Klimaanlagen, die bekanntlich mit Fluor- oder Chlorfluorkohlenwasserstoffen betrieben werden. Außerdem ist die Verbindung I ein wertvolles Zwischenprodukt für die Herstellung weiterer Produkte, beispielsweise für die Herstellung von 2-Chlor-2-trifluormethyl-decafluorpentan, von 3-Chlor-2-trifluormethyl-decafluorpentan und von Perfluor-(2-methyl-pentan), die durch katalytische Fluorierung mit Fluorwasserstoff erhalten werden können.

Aus Literaturangaben ist es bekannt, daß die Reaktivität von Oligomeren des Hexafluorpropens gegenüber Fluor mit zunehmender Verzweigung des Kohlenstoffgerüsts und steigendem Molekulargewicht drastisch abnimmt. Chlorierungen solcher oligomerer sind bisher nicht bekannt geworden. Es ist

daher überraschend, daß die Addition von Chlor an Perfluor-2-methyl-2-penten nach dem erfindungsgemäßen Verfahren so glatt und auch so quantitativ durchzuführen ist.

Die Erfindung wird durch die folgenden Beispiele näher erläutert.

Beispiel 1

Die Versuchsapparatur besteht aus einem zylindrischen, ca. 2 l fassenden Mehrhals-Bestrahlungskolben aus ®DURAN-Glas mit einem Durchmesser von 80 mm. Das Glasgefäß ist mit einem Innenthermometer, einem mit festem Kohlendioxid gefüllten Kondensor sowie mit einem am Boden des Gefäßes befindlichen Ablaßhahn ausgestattet. Ein Gaseinleitungsrohr für II reicht bis in das untere Drittel des Gefäßes, jedoch nicht bis zum Boden. Das Gaseinleitungsrohr für Chlor geht bis zum Boden des Gefäßes. Außerhalb des Glasgefäßes sind zwei 300 Watt ®ULTRA-VITALUX-Lampen der Firma Osram angebracht, von denen eine auf das untere Drittel und eine auf das mittlere Drittel des Bestrahlungsgefäßes gerichtet ist. Diese Lampen sorgen gleichzeitig für die erforderliche Heizung. Der obere Abgang des Kondensors ist mit einer tiefgekühlten kondensationsfalle verbunden, in der die durch den Kondensor eventuell nicht zurückgehaltenen Substanzen aufgefangen werden.

Elementares Chlor wird einer handelsüblichen Stahlflasche entnommen, mit konzentrierter Schwefelsäure getrocknet und in den Bestrahlungskolben eingeleitet, wobei die Dosierung durch einen Strömungsmesser erfolgt.

Zur Photochlorierung werden 2 000 g (6,67 Mol) II im Bestrahlungsgefäß vorgelegt und zunächst auf eine Temperatur von 45 bis 50 °C durch die eingeschalteten Bestrahlungslampen aufgeheizt. Nach Erreichen dieser Temperatur wird elementares Chlor eingeleitet und zwar so schnell, wie es bei der Umsetzung verbraucht wird. Wenn die Zugabe von Chlor zu rasch erfolgt, tritt starker Chlor-Rückfluß am Kondensor ein und zurückfließendes Chlor kühlt den Kolbeninhalt stark ab ; damit ist der Umsatz pro Zeiteinheit am Chlor noch geringer. Bei Temperaturen zwischen 20 und 50 °C gelingt es innerhalb von 22 Stunden insgesamt 260 g (3,66 Mol) Chlor umzusetzen ; innerhalb weiterer 6,5 Stunden lassen sich bei Temperaturen über 50 °C weitere 218 g (3,07 Mol) Chlor umsetzen. Das Destillationsprodukt wird über den Ablaßhahn am Boden des Gefäßes erst nach Beendigung der Reaktion entnommen. Anschließend wird das entstandene Rohprodukt gaschromatographisch untersucht. Dabei ergab sich folgende Zusammensetzung :

| | |
|---|---|
| $(CF_3)_2CCl—CClF—CF_2—CF_3$ | 97,8 % |
| $(CF_3)_2C = CF—CF_2—CF_3$ | 0,2 % |
| $C_{12}Cl_2F_{24}$-Isomerengemisch | 1,8 % |
| Sonstige | 0,2 % |

Zur Aufarbeitung wird das Rohprodukt destilliert ; dabei erhält man I als farblose, wasserklare Flüssigkeit, die bei 112,5°/1 bar siedet. Ausbeute : 2 344 g, entsprechend 94,9 % der Theorie, bezogen auf umgesetztes II. Zu einem Anteil von ca. 2 % erhält man ein bei 220 bis 225° siedendes Gemisch, bestehend aus verschiedenen $C_{12}Cl_2F_{24}$-Isomeren, die durch radikalische Dimerisierung von II entstehen.

Die Identifizierung der Verbindung I erfolgt durch Elementaranalyse, bei der für $C_6Cl_2F_{12}$ (MW 370,95) folgende Werte ermittelt wurden : C 19,35 % (berechnet : 19,43 %), Cl 19,5 % (ber. : 19,11 %) und F 61,45 % (ber. : 61,46 %).

Die Bestimmung der Dichte von I ergibt 1,830 g/cm³ bei 20 °C ; der Erstarrungspunkt liegt bei – 134 °C, der Brechungsindex bei $n_D^{20\,°C} = 1{,}323$.

Das Infrarotspektrum (flüss. Phase, kapillarer Film) zeigt die erwarteten Hauptbanden im Bereich von 1 300 bis 1 150 $cm^{-1}$ für $\upsilon_{C-F}$, bei 955 $cm^{-1}$ für $\upsilon_{C-C}$ sowie bei 733 und 695 $cm^{-1}$ für $\upsilon_{C-Cl}$.

Das Massenspektrum (durch Elektronenstoß-Ionisierung gemessen) enthält die charakteristischen Bruchstücke 335 m/e für $(Molekül-Cl)^+$ mit Intensität ca. 1 %, 251 m/e für $(Molekül-C_2F_5)^+$ mit 10 %, 185 m/e für $(CF_3CF_2CFCl)^+$ und/oder $((CF_3)_2CCl)^+$ mit 55 % und 69 m/e für $(CF_3)^+$ mit 100 % Intensität.

Im $^{19}F$-NMR-Spektrum treten aufgrund des asymmetrischen $C_3$-Atoms fünf statt vier kernmagnetisch verschiedene Fluorkerntypen auf ; die Resonanzsignale mit entsprechender Aufspaltung liegen bei $\delta = -121{,}7$ ppm (CF), – 116,6 ppm ($CF_2$), – 78,0 ppm ($CF_3$) sowie bei – 66,0 und – 65,2 ppm für jeweils eine der $CF_3$-Gruppen in $(CF_3)_2C$, bezogen auf $CCl_3F$ als internem Standard.

Beispiel 2

Die Versuchsanordnung aus Beispiel (1) wird mit einem weiteren Einleitungsrohr (für die Zuführung von II) augestattet, das wie das Chlor-Einleitungsrohr bis in das untere Drittel des Bestrahlungsgefäßes, nicht jedoch bis auf dessen Boden reicht. In diesem Beispiel wird II in einem Tropftrichter, der mit dem zusätzlich eingebauten Einleitungsrohr verbunden ist, flüssig vorgelegt, nicht jedoch im Bestrahlungskolben.

Im Bestrahlungskolben werden statt dessen 1 750 g (4,72 Mol) 2,3-Dichlor-2-trifluormethyl-nonafluorpentan (I) vorgelegt, wird gemäß Beispiel (1) hergestellt wurde.

Zur Photochlorierung von II wird das vorgelegte I auf eine Temperatur von 105 °C gebracht.

Anschließend werden bei Temperaturen von 105 °C bis 75 °C innerhalb von 6,5 h insgesamts 2 466 g (8,22 Mol) Perfluor-(2-methyl-2-penten) (II) und 610 g (8,59 Mol) Chlor, entsprechend einem Molverhältnis von II : $Cl_2$ wie 1 : 1,045 Mol, kontinuierlich und gleichmäßig mit einer Geschwindigkeit von ca. 1,26 Mol II/h und ca. 1,32 Mol $Cl_2$/h eingeleitet. Die Belichtung erfolgt dabei wie in Beispiel 1 angegeben. Während des Versuchs wird durch den am Boden befindlichen Ablaßhahn kontinuierlich I herausgeschleust mit einer Geschwindigkeit von ca. 450 bis 470 g/h. Dabei bleibt das Volumen des Kolbens etwa konstant.

Die gaschromatographische Untersuchung des erhaltenen Rohproduktes ergibt folgende Zusammensetzung :

| | |
|---|---|
| $(CF_3)_2CCl—CClF—CF_2—CF_3$ | 98,6 % |
| $(CF_3)_2C = CF—CF_2—CF_3$ | 1,1 % |
| $C_{12}Cl_2F_{24}$-Isomere | 0,3 % |

Die nachfolgende Destillation ergibt eine Hauptfraktion bei 112,5 °C mit einem Gewicht von 2 988 g (8,05 Mol) I, entsprechend einer Ausbeute von 99,0 % d. Th. bezogen auf umgesetztes II.

## Ansprüche

1. 2,3-Dichlor-2-trifluormethyl-1,1,1,3,4,4,5,5,5-nonafluorpentan.
2. Verfahren zur Herstellung der Verbindung von Anspruch 1, dadurch gekennzeichnet, daß man Perfluor-(2-methyl-2-penten) mit elementarem Chlor in Gegenwart von energiereichem Licht und in flüssigem 2,3-Dichlor-2-trifluormethyl-1,1,1,3,4,4,5,5,5-nonafluorpentan als Lösungsmittel bei Temperaturen von 20-300 °C umsetzt.
3. Verfahren nach Anspruch 2, dadurch gekennzeichnet, daß man Chlor und Perfluor-(2-methyl-2-penten) kontinuierlich umsetzt.
4. Verfahren nach Anspruch 2, dadurch gekennzeichnet, daß man bei Temperaturen von 50 bis 200 °C arbeitet.

## Claims

1. The compound 2,3-dichloro-2-trifluoromethyl-1,1,1,3,4,4,5,5,5-nonafluoropentane.
2. A process for the manufacture of the compound claimed in claim 1, which comprises reacting perfluoro-(2-methyl-2-pentene) with elementary chlorine in the presence of light rich in energy in liquid 2,3-dichloro-2-trifluoromethyl-1,1,1,3,4,4,5,5,5-nonafluoropentane as solvent at a temperature of from 20 to 300 °C.
3. The process of claim 2, which comprises reacting chlorine and perfluoro-(2-methyl-2-pentene) continuously.
4. The process of claim 3, which comprises working at a temperature of from 50 to 200 °C.

## Revendications

1. Dichloro-2,3 trifluorométhyl-2 nonafluoro-1,1,1,3,4,4,5,5,5 pentane.
2. Procédé de préparation du composé selon la revendication 1, procédé caractérisé en ce qu'on fait réagir le perfluoro méthyl-2 pentène-2 avec du chlore élémentaire, à des températures de 20 à 300 °C, en présence d'un rayonnement de haute énergie et dans du dichloro-2,3 trifluorométhyl-2 nonafluoro-1,1,1,3,4,4,5,5,5 pentane liquide comme solvant.
3. Procédé selon la revendication 2, caractérisé en ce qu'on fait réagir le chlore et le perfluoro méthyl-2 pentène-2 en continu.
4. Procédé selon la revendication 2, caractérisé en ce qu'on opère à des températures de 50 à 200 °C.